# EUROPEAN PATENT APPLICATION

(11) **EP 4 324 486 A1**
(43) Date of publication of application: **21.02.2024**
(21) Application number: 22788248.7
(22) Date of filing: 18.02.2022
(51) Int. Cl.: A61K 48/00, A61K 31/713, A61P 35/00, G01N 33/50, C12N 15/113, C07K 16/28

(54) **USE OF ODORANT RECEPTOR FOR SUPPRESSING LACTATE-INDUCED M2-TYPE DIFFERENTIATION OF TUMOR-ASSOCIATED MACROPHAGES AND TUMOR GROWTH**

(30) Priority: 16.04.2021 KR 20210049718
(71) Applicant: Kyungpook National University Industry-Academic Cooperation Foundation, Daegu 41566 (KR); Daegu Gyeongbuk Institute Of Science and Technology, Daegu 42988 (KR)
(72) Inventor: LEE, Byung-Heon, Daegu 42038 (KR); KOO, Jaehyung, Dalseong-gun Daegu 42988 (KR); SRI MURUGAN, Poongkavithai Vadevoo, Daegu 41944 (KR)
(74) Representative: dompatent von Kreisler Selting Werner - Partnerschaft von Patent- und Rechtsanwälten mbB
(86) International application number: PCT/KR2022/002398
(87) International publication number: WO 2022/220390

(57) **Abstract**

The present invention relates to use of an odorant receptor for suppressing the lactate-induced M2-type differentiation of tumor-associated macrophages and tumor growth. In particular, the inventors of the present invention assumed that lactate-activated OR Olfr78 would function as a macrophage lactate sensor, and confirmed whether Olfr78 on bone marrow-derived macrophages (BMDMs) detect tumor-derived lactate and lactate-induced M2 polarization. The inventors of the present invention attempted to identify factors detected by Olfr78 in tumor conditioned media (TCM), and discovered that lactate in TCM and acetic acid under some conditions are major factors involved in Olfr78-mediated generation of tumor-friendly M2-TAMs. Finally, it was found that Olfr78 deficiency inhibited tumor progression and metastasis and improved antitumor immunity in vivo (in vivo). According to the present invention, Olfr78-lactate interaction plays a major role in tumor progression, and thus, targeting of the Olfr78-lactate axis can be effectively used in targeted cancer therapy.

## Description

### Technical Field

The present disclosure relates to a use of odorant receptors for suppressing lactate-induced M2 type differentiation of tumor-associated macrophages and tumor growth.

### Background Art

Odorant receptors (ORs) are the largest subfamily of G protein-coupled receptors (GPCRs), accounting for 388 of 785 human GPCRs and 1,037 of 1,678 mouse GPCRs. GPCRs are a major type of drug target and have been extensively studied. However, previous studies have focused on the functions of ORs in the nose, and investigations of their ectopic expression and functions in nonolfactory tissues are lacking. The development of next-generation sequencing techniques, such as bulk and single-cell RNA sequencing, has made it possible to detect and analyze genes that are lowly expressed or expressed in certain cell types. Consequently, research into the functions of ectopic ORs is growing rapidly, and such ORs have been suggested as potential drug targets. Nevertheless, studies of ectopic ORs as drug targets in tumors are limited due to the lack of identification and characterization of ORs in pathophysiological conditions in vivo, such as in the tumor microenvironment (TME).

Tumor-associated macrophages (TAMs) are one of the main components of the TME and promote tumor progression, angiogenesis, invasion, metastasis, and immunosuppression. Macrophages differentiate into protumoral M2-TAMs in response to stimuli such as lactate, interleukin (IL)-4, IL-13, IL-10, macrophage colony-stimulating factor, and corticosteroids in the TME. Lactate produced in the tumor landscape is transported by monocarboxylate transporters expressed in various cell types and used as a carbon source by lactate dehydrogenase. It also acts as a signaling molecule that binds to GPCRs. Lactate affects many cell types, including macrophages, effector T cells, and regulatory T cells (Tregs), in the TME. Among these, macrophage polarization to generate M2-TAMs plays a crucial role in immunomodulation in the TME, which in turn promotes tumor progression and metastasis. However, crosstalk between lactate and macrophages leading to generation M2-TAMs that enhance tumor progression remains poorly defined.

### Disclosure of the Invention

### Technical Goals

An object of the present disclosure is to provide a pharmaceutical composition for preventing or treating cancer, including an expression or activity inhibitor of odorant receptor Olfr78 or OR51E2 as an active ingredient.

In addition, another object of the present disclosure is to provide a method of screening an anticancer drug, including selecting a test substance in which a decree of expression or activity of odorant receptor Olfr78 or OR51E2 is reduced in isolated macrophages.

In addition, another object of the present disclosure is to provide a method of screening an anticancer drug, including selecting a test substance in which a degree of binding of odorant receptor Olfr78 or OR51E2 and Gpr132 is reduced in isolated macrophages.

In addition, another object of the present disclosure is to provide a reagent composition for inducing lactate- or acetate-induced M2 polarization of macrophages in vitro, including odorant receptor Olfr78 or OR51E2 as an active ingredient, and a method of inducing lactate- or acetate-induced M2 polarization of macrophages using the same.

### Technical Solutions

In order to achieve the above objects, the present disclosure provides a pharmaceutical composition for preventing or treating cancer, including an expression or activity inhibitor of odorant receptor Olfr78 or OR51E2 as an active ingredient.

In addition, the present disclosure provides a method of screening an anticancer drug including: (1) contacting test substances with isolated macrophages; (2) measuring a degree of expression or activity of odorant receptor Olfr78 or OR51E2 in the macrophages contacted with the test substances; and (3) selecting a test substance with a reduced degree of the expression or activity of Olfr78 or OR51E2 compared to a control sample.

In addition, the present disclosure provides a method of screening an anticancer drug including: (1) contacting test substances with isolated macrophages; (2) measuring a degree of binding of odorant receptor Olfr78 or OR51E2 and Gpr132 in the macrophages contacted with the test substances; and (3) selecting a test substance with a reduced degree of the binding of Olfr78 or OR51E2 and Gpr132 compared to a control sample.

In addition, the present disclosure provides a reagent composition for inducing lactate- or acetate-induced M2 polarization of macrophages in vitro, including odorant receptor Olfr78 or OR51E2 as an active ingredient.

In addition, the present disclosure provides a method of inducing lactate- or acetate-induced M2 polarization of macrophages, including treating macrophages with odorant receptor Olfr78 or OR51E2 in vitro.

### Advantageous Effects

The present disclosure relates to a use of odorant receptors for suppressing lactate-induced M2 type differentiation of tumor-associated macrophages and tumor growth, and specifically, the present inventors hypothesized that the lactate-activated OR Olfr78 functions as a macrophage lactate sensor, and sought to determine whether Olfr78 on bone marrow-derived macrophages (BMDMs) senses tumor-derived lactate and mediates lactate-induced M2 polarization. For the study of the role of Olfr78 as a lactate sensor on macrophages, the inventors used Olfr78^{-/-} mice. In addition, the inventors sought to identify factors in tumor-conditioned media (TCM) that are sensed by Olfr78 and found that lactate and, under some conditions, acetate in TCM were the main factors involved in Olfr78-mediated generation of protumoral M2-TAMs. Furthermore, it was found that Olfr78 formed a heterodimer with Gpr132 that mediates lactate-induced M2 phenotype of TAMs. Finally, it was demonstrated that Olfr78 deficiency inhibited tumor progression and metastasis and favored antitumor immunity in vivo. According to the present disclosure, the Olfr78-lactate interaction plays a key role in tumor progression, and thus targeting the Olfr78-lactate axis may be applicable for targeted cancer therapy.

### Brief Description of Drawings

FIG. 1 shows results indicating that Olfr78 mediates lactate-induced M2 polarization of mouse BMDMs.
FIG. 2 shows results indicating that lactate induces M2 polarization of THP-1 human monocytes.
FIG. 3 shows results indicating that OR51E2 mediates lactate-induced M2 polarization of THP-1 human monocytes.
FIG. 4 shows results indicating that lactate and acetate, but not butyrate, are key molecules in TCM-induced M2 polarization in mouse BMDMs.
FIG. 5 shows results indicating that lactate is a key molecule in TCM-induced M2 polarization in THP-1 human monocytes.
FIG. 6 shows results indicating that Olfr78 forms a heterodimer with Gpr132 to mediate lactate-induced M2 polarization in mouse BMDMs.
FIG. 7 shows results indicating that deficiency of Olfr78 enhances anti-tumor immunity and inhibits progression and metastasis of LLC mouse lung tumors.
FIG. 8 shows results indicating that deficiency of Olfr78 alters the immune cell population in the spleen and enhances anti-tumor immunity in mice with LLC mouse lung tumors.
FIG. 9 shows results indicating that deficiency of Olfr78 inhibits the progression and metastasis of EO771 mouse breast tumors.
FIG. 10 shows results indicating that deficiency of macrophages using clodronate liposomes inhibits tumor progression in mice with LLC syngeneic lung tumors.
FIG. 11 shows results indicating that, on the basis of TCGA analysis, patients with low OR51E2 expression have a longer survival rate compared to patients with high OR51E2 expression.

### Best Mode for Carrying Out the Invention

The present disclosure provides a pharmaceutical composition for preventing or treating cancer, including an expression or activity inhibitor of odorant receptor Olfr78 or OR51E2 as an active ingredient.

In detail, the expression inhibitor of Olfr78 or OR51E2 may be any one selected from the group consisting of antisense nucleotides, small interfering RNAs (siRNAs), and short hairpin RNAs (shRNAs) that complementarily bind to mRNA of Olfr78 or OR51E2 gene, and the activity inhibitor of Olfr78 or OR51E2 may be any one selected from the group consisting of low molecular weight compounds, peptides, peptide mimetics, aptamers, antibodies, and natural products that specifically bind to Olfr78 or OR51E2 protein, but are not limited thereto.

Preferably, the composition may inhibit lactate- or acetate-induced M2 polarization of macrophages.

Preferably, the cancer may be lung cancer or breast cancer, but is not limited thereto.

The pharmaceutical composition of the present disclosure may be prepared using a pharmaceutically suitable and physiologically acceptable adjuvant in addition to the active ingredient, wherein solubilizers such as an excipient, disintegrant, sweetener, binder, coating agent, swelling agent, antifriction agent, lubricant, or flavoring agent may be used as the adjuvant. The pharmaceutical composition of the present disclosure may preferably be prepared as a medicinal composition including one or more types of pharmaceutically acceptable carriers in addition to the active ingredient for administration. In a composition prepared in a liquid solution, acceptable pharmaceutical carriers including saline, sterile water, Ringer's solution, buffer saline, albumin injection solution, dextrose solution, maltodextrin solution, glycerol, ethanol and one or more components thereof that are sterile and suitable in the body may be mixed and used, and other conventional additives such as antioxidants, buffers, and bacteriostatic agents may be added as needed. In addition, diluents, dispersants, surfactants, binders, and lubricants may be additionally added to prepare injectable formulations such as aqueous solutions, suspensions, and emulsions, pills, capsules, granules, or tablets.

The pharmaceutical preparation form of the pharmaceutical composition of the present disclosure may be granules, acids, coated tablets, tablets, capsules, suppositories, syrups, juices, suspensions, emulsions, drip agents or injectable liquid, and slow-release preparation of active compounds. A medicinal composition of the present disclosure may be administered in a conventional manner through intravenous, intra-arterial, intraperotoneal, intramuscular, intra-arterial, intraperotoneal, intrasternal, percutaneous, intranasal, inhalation, topical, rectal, oral, intraocular, or intradermal routes. The effective dose of the active ingredient in the medicinal composition of the present disclosure refers to an amount required for preventing or treating a disease. Thus, it may be adjusted by various factors including the type of a disease, the severity of the disease, the type and content of the active ingredient and other components included in the composition, the type of formulation and the patient's age, weight, general health status, gender, and diet, administration time, administration route and secretion rate of the composition, duration of treatment, and drugs used in combination with.

In addition, the present disclosure provides a method of screening an anticancer drug including: (1) contacting test substances with isolated macrophages; (2) measuring a degree of expression or activity of odorant receptor Olfr78 or OR51E2 in the macrophages contacted with the test substances; and (3) selecting a test substance with a reduced degree of the expression or activity of Olfr78 or OR51E2 compared to a control sample.

In addition, the present disclosure provides a method of screening an anticancer drug including: (1) contacting test substances with isolated macrophages; (2) measuring a degree of binding of odorant receptor Olfr78 or OR51E2 and Gpr132 in the macrophages contacted with the test substances; and (3) selecting a test substance with a reduced degree of the binding of Olfr78 or OR51E2 and Gpr132 compared to a control sample.

Preferably, the composition may inhibit lactate- or acetate-induced M2 polarization of macrophages.

Preferably, the anticancer drug may be an anticancer drug for lung cancer or breast cancer, but is not limited thereto.

The term "test substance" as used herein in reference to the screening method of the present disclosure refers to an unknown candidate substance used in screening to test whether it affects the expression level of a gene, the expression or activity of a protein, or the binding between proteins. The samples include, but are not limited to, chemicals, nucleotides, antisense-RNAs, small interference RNAs (siRNAs), and extracts of natural products.

In addition, the present disclosure provides a reagent composition for inducing lactate- or acetate-induced M2 polarization of macrophages in vitro, including odorant receptor Olfr78 or OR51E2 as an active ingredient.

In addition, the present disclosure provides a method of inducing lactate- or acetate-induced M2 polarization of macrophages, including treating macrophages with odorant receptor Olfr78 or OR51E2 in vitro.

### Modes for Carrying Out the Invention

Hereinafter, the present disclosure will be described in more detail through example embodiments. These example embodiments are only for the purpose of describing the present disclosure more specifically, and it would be obvious to those skilled in the art that the scope of the present disclosure is not limited by these example embodiments according to the gist of the present disclosure.

### <Experimental Example>

The following experimental examples are intended to provide experimental examples commonly applied to each example embodiment according to the present disclosure.

### 1. Cell culture

LLC lung and B16F10 melanoma mouse tumor cell lines and the MDA-MB231 human breast tumor cell lines were cultured in Dulbecco's modified Eagle's medium (DMEM; HyClone, South Logan, UT). The 4T1 mouse breast tumor cell lines and MCF7 breast and A549 lung human tumor cell lines were cultured in RPMI medium (HyClone). All cell cultures were supplemented with 10% fetal bovine serum (FBS; ThermoFisher, Waltham, MA) and maintained at 37°C in a humidified atmosphere containing 5% CO₂.

### 2. M1 and M2 Polarization of BMDMs.

BMDMs were isolated from tibias and femurs of C57BL6 or Balb/c mice and cultured in DMEM supplemented with 10 ng/mL macrophage colony-stimulating factor (Gibco, Carlsbad, CA) and 10% FBS for 7 d. The culture medium was changed every other day. For M1 polarization, BMDMs were cultured with 20 ng/mL recombinant mouse IFN-γ (R&D Systems) plus 100 ng/mL LPS for 24 to 48 h. For M2 polarization, BMDMs were cultured with 20 ng/mL recombinant mouse IL-4 for 24 to 48 h. For lactate-mediated M2 polarization, BMDMs were treated with 10 mM lactate (Sigma-Aldrich, St. Louis, MO) at 37°C in a humidified atmosphere containing 5% CO₂ for 24 to 48 h.

### 3. Flow cytometry

Anti-CD11b, anti-CD3, anti-CD4, anti-CD8, anti-CD86, anti-F4/80, anti-CD206, anti-CD163, and anti-Gr1 antibodies were purchased from BioLegend (San Diego, CA). An anti-Olfr78 antibody was purchased from LSBio (Seattle, WA). Anti-CD206 and anti-CD86 antibody was purchased from Santa Cruz Biotechnology (Dallas, TX). Cells were fixed with 4% paraformaldehyde and then reacted with an untagged primary antibody or a dye-tagged antibody at 4°C for 30 min in the dark. Cells treated with an untagged primary antibody (anti-Olfr78, anti-CD206, and anti-CD86) were washed and reacted with a dye-tagged secondary antibody. At least 10,000 or more events were analyzed using a flow cytometer (BD Bioscience, San Jose, CA). Data were evaluated using CellQuest Pro software (BD Biosciences). To prepare cell suspensions from tumor tissues, freshly excised tumors were fragmented into several pieces, minced into 2-3 mm³ pieces, and reacted with collagenase D (Roche, Basel, Switzerland) and DNase (Sigma-Aldrich) at 37 °C for 40 min. The tissue samples were filtered through a 100 µm cell strainer (Falcon, Corning, NY) to collect digested cells. Collected cells were resuspended in phosphate-buffered saline containing FBS and subjected to flow cytometry.

### 4. Quantitative reverse transcription-polymerase chain reaction (qRT-PCR)

Cells were lysed using QIAzol lysis reagent (Qiagen, Hilden, Germany). RNA was isolated from cell lysates using an miRNeasy Mini Kit (Qiagen) and subjected to qRT-PCR. Primers targeting Arg1, IL-10, IL-4, TGF-β, IL-12p40, IFN-γ, and β-actin were obtained from Bioneer Inc. (Daejeon, Korea). cDNA was synthesized using a PrimeScript 1st cDNA Synthesis Kit (Takara, Shiga, Japan). qPCR using SYBR Green (Qiagen) was performed on a real time cycler (Bio-Rad, Hercules, CA). β-Actin was used as an endogenous control. Data used to determine relative expression were analyzed according to the Livak method. The primer sequences are listed in Table 1.

**TABLE 1**

| **Target** | **Forward Primer(5'-3')** | **Reverse Primer-(3'-5')** |
|---|---|---|
| *Arg1* | GAACACGGCAGTGGCTTTAAC | TGCTTAGCTCTGTCTGCTTTGC |
| *IL-10* | GGTTGCCAAGCCTTATCGGA | ACCTGCTCCACTGCCTTGCT |
| *IL-4* | ACAGGAGAAGGGACGCCAT | GAAGCCCTACAGACGAGCTCA |
| *TGF-β* | TGACGTCACTGGAGTTGTACGG | GGTTCATGTCATGGATGGTGC |
| *IL-12p40* | GGAAGCACGGCAGCAGAATA | AACTTGAGGGAGAAGTAGGAATGG |
| *IFN-γ* | TCAAGTGGCATAGATGTGGAAGAA | TGGCTCTGCAGGATTTTCATG |
| *Olfr78* | CGCTGCTGTCCTCAACAATA | AAGCCAGTCGCTTGATCAGT |
| *β-actin* | CAAAAGCCACCCCCACTCCTAAGA | GCCCTGGCTGCCTCAACACCTC |
| *OR51E2* | TCATATTTGCAAGCTCGGCC | TGAGGTCACACTGGCAGTAG |

### 5. Immunofluorescence microscopy

Cells (1 × 10⁵ cells per well in a 4-well chamber) were fixed with 4% paraformaldehyde and reacted with antibodies against Olfr78 and Gpr132 (1:100 dilution; Santa Cruz Biotechnology) at 24°C for 1 h or at 4°C for 16 h. Cells were then reacted with fluorophore-tagged secondary antibodies at 24°C for 1 h. Cells were stained with 4',6-diamidino-2-phenylindole (DAPI) to label nuclei, mounted with Prolong Gold anti-fade mounting reagent (Life Technologies, Eugene, OR), and observed under a confocal microscope.

### 6. Measurement of lactate and acetate concentrations and measurement of cytokine concentrations through enzyme-linked immunosorbent assays (ELISAs)

The concentrations of lactate and acetate in TCM of cells cultured at 80% confluency were measured using lactate and acetate colorimetric assay kits (BioVision, Milpitas, CA). Briefly, 50 µL of TCM was mixed with lactate or acetate enzyme mix and the probe according to the manual and reacted for 30 min at room temperature. The concentrations of cytokines in the conditioned medium of macrophages were measured using cytokine ELISA kits (R&D Systems). Briefly, 100 µL of conditioned medium was added to plates precoated with antibodies against IL-4, IL-10, and TGF-β. Samples were reacted with corresponding antibodies conjugated to biotin and then with horseradish peroxidase-labeled streptavidin. Absorbance at 570 nm was measured using a microplate reader. The concentrations of lactate, acetate, and cytokines were calculated from the standard curves.

### 7. Proximity ligation assay (PLA)

BMDMs were reacted with a rabbit anti-Olfr78 antibody (LifeSpan Biosciences, Seattle, WA) and a mouse anti-Gpr132 antibody (Sigma-Aldrich) at 24°C for 1 h. Rabbit IgG and mouse IgG were used as controls. Cells were then reacted with an anti-rabbit MINUS PLA probe and an anti-mouse PLUS PLA probe at 37°C for 60 min. After washing, cells were reacted with ligation stocks and ligase mixtures at 37°C for 30 min and then with amplification stocks and polymerase mixtures at 37°C for 100 min. Samples were mounted with Duolink in situ mounting medium (Sigma-Aldrich) and observed under a confocal microscope (Nanoscope, Daejeon, Korea).

### 8. Coimmunoprecipitation assays and western blot analysis

For coimmunoprecipitation assays, cells were lysed with M-PER Mammalian Protein Extraction Reagent (ThermoFisher Scientific, Waltham, MA). Cell lysates were reacted with 2 µg of an antibody against Olfr78 or Gpr132 at 4°C overnight and then with 100 µl of protein A-agarose beads (ThermoFisher Scientific) at 4°C for 4 h with agitation. The beads were boiled, and the eluates were subjected to Western blotting with an antibody against Olfr78 or Gpr132. 40 µg of protein was electrophoresed on a 10% SDS-PAGE gel and transferred to a membrane (MilliPore, Burlington, MA). The membrane was reacted with antibodies against iNOS (Abeam, Cambridge, UK), Arg1 (Abeam), and β-actin (Santa Cruz Biotechnology) at 4°C overnight followed by secondary antibody at room temperature for 2 h, and then exposed to a chemiluminescence substrate (ThermoFisher Scientific). Signals were visualized using an LAS1000 enhanced chemiluminescence detection system (Fuji Film, Minato, Tokyo).

### 9. Luciferase assay

Luciferase assays were performed using the Dual-Glo^{™} Luciferase Assay System (Promega, Madison, WI). Briefly, Mock-, Olfr78-, Gpr132-, or Olfr78/Gpr132 transiently transfected Hana3A cells were stimulated with various concentrations of sodium lactate diluted in CD293 medium at 37°C for 4 h. The activity of firefly luciferase was normalized against that of Renilla luciferase. Luminescence was measured on a SpectraMax L microplate reader (Molecular Devices, San Jose, CA).

### 10. Transfection and Expression of Olfr78

The lucy-flag-rho-Olfr78-pME18s expression vector was constructed and transfected into Olfr78^{-/-} BMDMs. For transfection, cells (1 × 10⁵) were cultured in 100 µL of antibiotic-free OptiMEM medium and transfected using 0.5 µg of the Olfr78 expression vector and 2 µL of Lipofectamine 2000 (ThermoFisher Scientific, Waltham, MA). Expression of Olfr78 was identified by immunofluorescence staining using antibodies against Flag and Olfr78.

### 11. siRNA transfection

siRNAs against OR51E2 and mouse Gpr132 were purchased from Bioneer and Dharmacon (Lafayette, CO), respectively. BMDMs (1 × 10⁵) were cultured in 100 µL of antibiotic-free Opti-MEM medium and transfected using 25 pmol siRNA and 2 µL of Lipofectamine RNAiMAX (ThermoFisher Scientific). Gene knockdown was detected by Western blot analysis.

### 12. Animal models

C57BL6 mice aged 6 to 8 wk were purchased from Orient Bio (Seongnam, Korea). Olfr78^{-/-} C57BL6 mice were provided by Dr. Jennifer Pluznick (Johns Hopkins University). Mice were cared for and maintained in accordance with the guidelines of the Institutional Animal Care and Use Committee of Kyungpook National University (Permission no. 2015-0017) or the Daegu Gyeongbuk Institute of Science and Technology. The LLC syngeneic lung tumor model was prepared by subcutaneously injecting 1×10⁶ LLC cells into the lower right flank of male mice. The EO771 syngeneic breast tumor model was prepared by implanting 1×10⁶ EO771 cells into the lower left mammary fat pad of female mice. At the end of treatment, mice were sacrificed for analysis of immune cells in tumors and the spleen or maintained to determine the survival rate.

### 13. Statistical analysis

Data was expressed as mean ± SD. All statistical analyses were performed using GraphPad Prism software (GraphPad Software, San Diego, CA). Statistical significance was determined by a one-way ANOVA with Tukey's multiple-comparison test or a two-way ANOVA with the Bonferroni multiple-comparison test. P < 0.05 was considered significant.

### <Example 1> Olfr78 senses lactate and mediates lactate-induced M2 polarization of macrophages

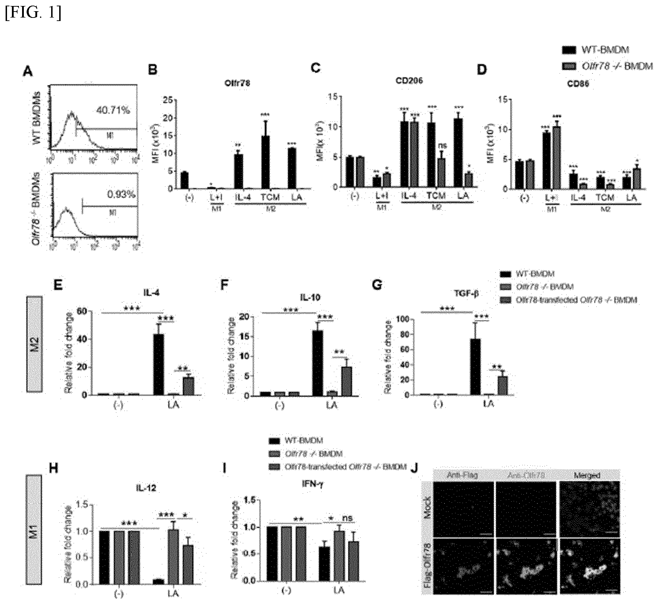

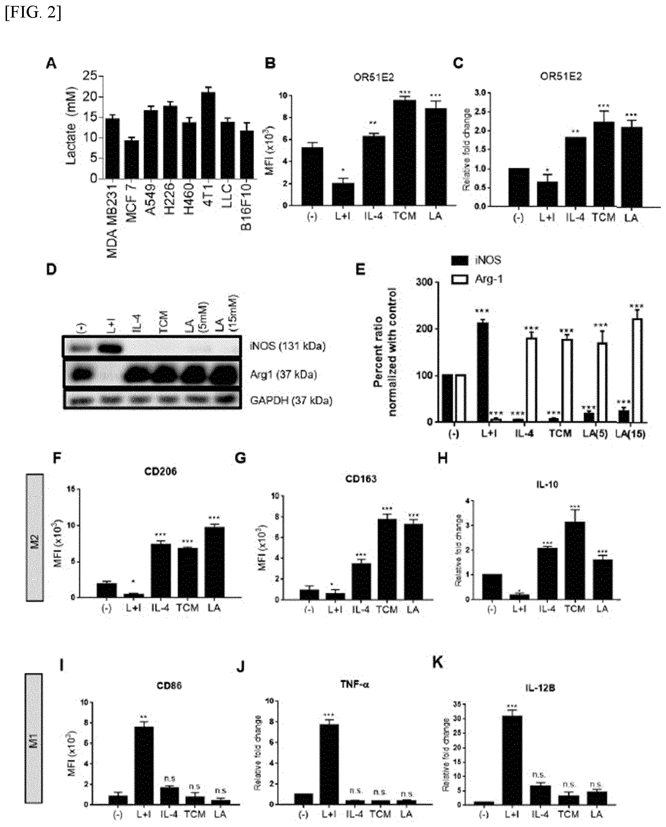

Lactate activates Olfr78 in a dose-dependent manner and has EC50 of 4 to 21 mM. To examine the involvement of Olfr78 in lactate-induced M2 polarization of macrophages, the present inventors conducted investigation on whether Olfr78 was expressed in BMDMs isolated from Olfr78^{+/+} (WT) and Olfr78^{-/-} mice. Approximately 41% and 1% of BMDMs isolated from WT and Olfr78^{-/-} mice expressed Olfr78, respectively (FIG. 1A). The concentration of lactate in TCM of human and mouse tumor cells was ~8 to 20 mM (FIG. 2A). In other words, the concentration of lactate produced in tumors is similar to the physiological concentration range of lactate that activates Olfr78. Next, the present inventors differentiated BMDMs into M1 and M2 macrophages to investigate whether the expression level of Olfr78 was regulated according to macrophage polarization. Lactate, TCM, and IL-4 were used to stimulate M2 polarization, while lipopolysaccharide (LPS) plus interferon (IFN)-γ (L+I) was used to stimulate M1 polarization. Olfr78 expression significantly increased (P < 0.01) upon differentiation of BMDMs into M2 macrophages in response to lactate, TCM, and IL-4 but significantly (P < 0.05) decreased upon L+I-induced M1 polarization (FIG. 1B), suggesting that Olfr78 is an M2 macrophage marker. To investigate the effect of Olfr78 on macrophage polarization, the present inventors measured the protein levels of M2 macrophage markers such as CD206 (FIG. 1C) and the mRNA levels of IL-4, IL-10, and TGF-β (FIGS. 1E-G), as well as M1 macrophage markers such as CD86 (FIG. 1D) and the mRNA levels of IL-12 and IFN-γ (FIG. 1H and 1I) when WT and Olfr78^{-/-} BMDMs were differentiated into M1 and M2 macrophages. The protein level of CD206 (FIG. 1C) and the mRNA levels of IL-4, IL-10, and TGF-β (FIGS. 1E-G) were dramatically (P < 0.001) higher and the protein level of CD86 (FIG. 1D) and the mRNA levels of IL-12 and IFN-γ (FIG. 1H and 1I) were significantly lower in lactate-treated WT BMDMs than in untreated BMDMs (-). Similarly, TCM and IL-4 treatment up-regulated CD206 and down-regulated CD86 in WT BMDMs, while L+I treatment elicited the opposite effects (FIG. 1C and FIG. 1D). However, lactate-treated Olfr78^{-/-} BMDMs did not exhibit up-regulation of M2-specific markers or down-regulation of M1-specific markers compared with untreated BMDMs (FIGS. 1C-I). IL-4 treatment still up-regulated CD206 (FIG. 1C) and down-regulated CD86 (FIG. 1D) in Olfr78^{-/-} BMDMs, suggesting that Olfr78 is required for lactate- and TCM-induced, but not IL-4-induced, M2 polarization of macrophages. Next, the present inventors restored Olfr78 expression in Olfr78^{-/-} BMDMs by transfecting them with a Flag-tagged Olfr78 expression vector. Immunostaining using anti-Flag and anti-Olfr78 antibodies demonstrated expression of exogenous Olfr78 in transfected Olfr78^{-/-} BMDMs (FIG. 1J). Exogenous Olfr78 expression in Olfr78^{-/-} BMDMs partly restored the lactate-induced changes in mRNA levels of cytokines compared with WT and Olfr78^{-/-} BMDMs (FIGS. 1E-I).

To examine the effect of ORs on M2 polarization of human macrophages, THP-1 human immortalized monocytes were treated with phorbol myristate acetate (PMA) and then with L+I, IL-4, TCM, or lactate. The protein (FIG. 2B) and mRNA (FIG. 2C) levels of OR51E2, a human analog of mouse Olfr78, in THP-1 cells significantly (P < 0.01) increased upon treatment with lactate, TCM, and IL-4 but decreased (P < 0.05) upon treatment with L+I, suggesting that OR51E2 was induced in M2-polarized human macrophages, similar to mouse Olfr78. Similar to IL-4 and TCM, lactate treatment increased the levels of M2 macrophage markers such as Arg1, CD206, CD163, and IL-10 (FIGS. 2D-H). On the other hand, it did not affect the levels of M1 macrophage markers such as CD86, TNF-α, and IL-12B (FIGS. 2I-K), and decreased iNOS levels compared with untreated cells (FIG. 2D and FIG. 2E).

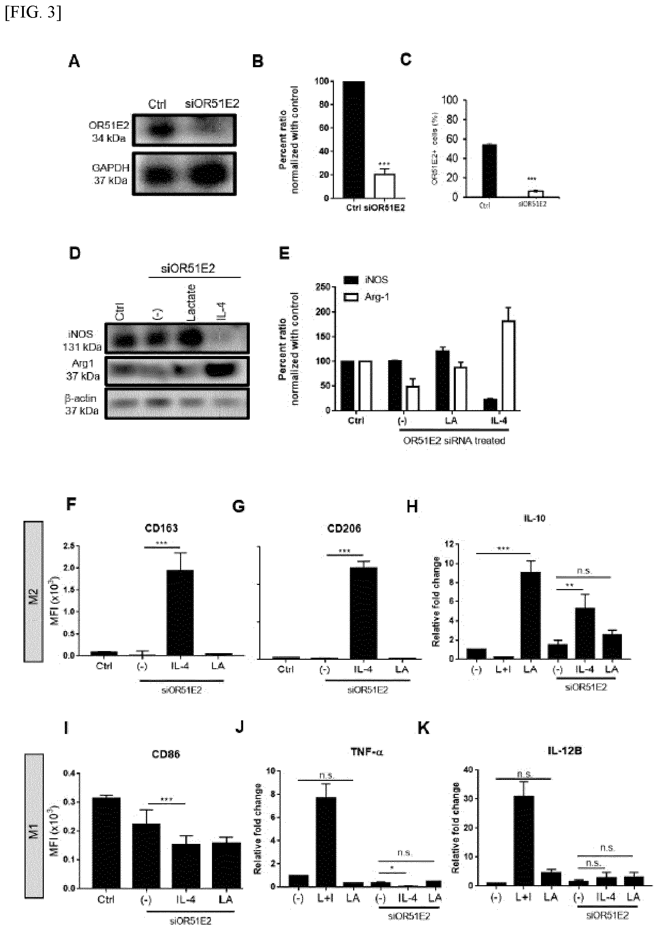

Next, the present inventors depleted OR51E2 in THP-1 cells. Transfection of an OR51E2-targeting siRNA decreased the OR51E2 protein level (FIG. 3A and FIG. 3B) and the percentage of OR51E2-expressing cells (FIG. 3C). Compared with untreated cells, lactate treatment did not up-regulate M2 markers such as Arg-1, CD163, CD206, and IL-10 (FIGS. 3D-H) or down-regulate M1 markers such as iNOS (FIG. 3D and FIG. 3E), CD86, TNF-α, and IL-12B (FIGS. 3I-K) in OR51E2-depleted THP1 cells. IL-4 treatment still up-regulated M2 markers (FIGS. 3D-H) and down-regulated M1 markers such as CD86 and TNF-α (FIG. 3I and FIG. 3J) in OR51E2-depleted THP1 cells. Taken together, these results suggest that OR51E2 is required for lactate- and TCM-induced, but not IL-4-induced, M2 polarization of macrophages.

### <Example 2> Lactate and acetate are the main factors in TCM responsible for Olfr78-mediated M2 macrophage polarization

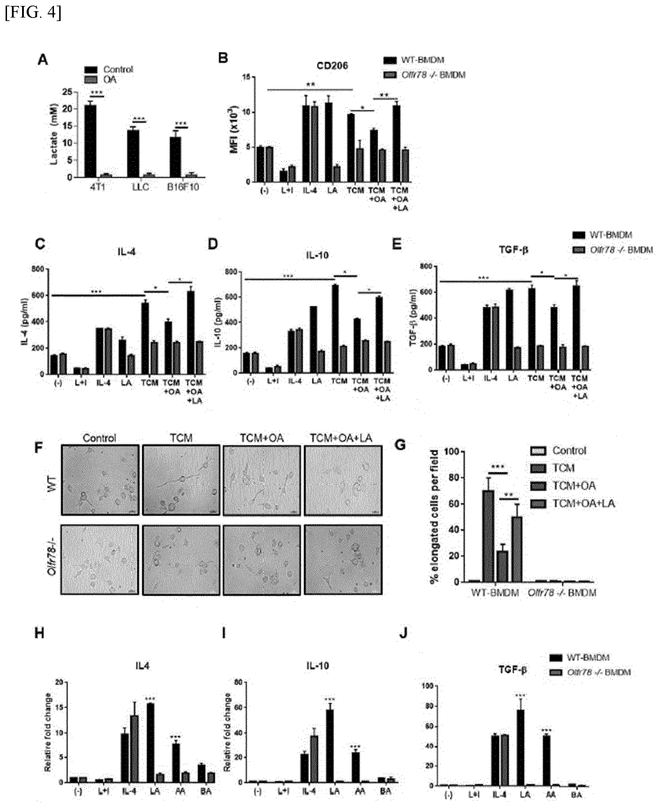

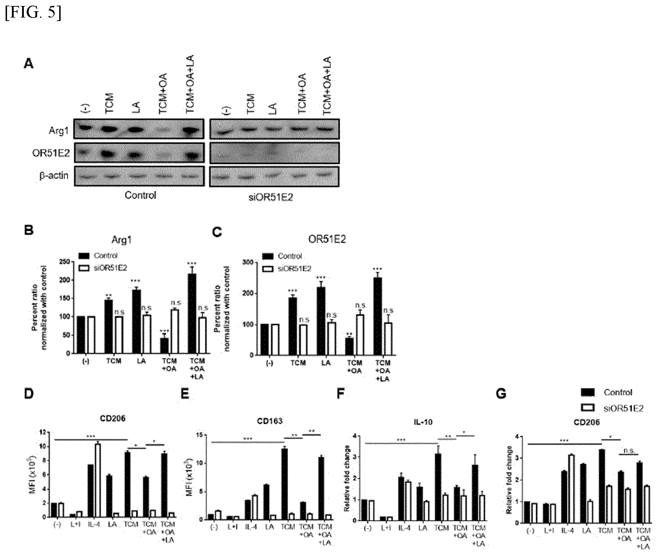

To determine, though lactate is known to be important in M2 macrophage polarization, whether lactate is the main factor in TCM responsible for Olfr78-mediated M2 macrophage polarization, the prevent inventors treated WT BMDMs with TCM of mouse tumor cell lines pretreated with oxamic acid (OA), an inhibitor of lactate dehydrogenase that prevents lactate production. Pretreatment with OA almost completely depleted lactate in TCM (FIG. 4A). Compared with untreated BMDMs, lactate- and TCM-treated WT BMDMs exhibited significant up-regulation (P < 0.001) of M2 polarization markers, such as CD206, IL-4, IL-10, and TGF-β (FIGS. 4B-E), and an increase (P < 0.001) in the percentage of cells with an elongated shape (FIG. 4F and FIG. 4G). Up-regulation of M2 markers was attenuated (P < 0.05) in WT BMDMs treated with lactate-depleted TCM (TCM+OA). Such changes in the levels of M2 markers were not observed in Olfr78^{-/-} BMDMs (FIGS. 4B-E). Addition of exogenous lactate to lactate-depleted TCM (TCM+OA+LA) restored (P < 0.05) M2 polarization of WT, but not of Olfr78^{-/-} BMDMs (FIGS. 4B-E). Through the experiments, the present inventors suggest that lactate is the main factor in TCM responsible for Olfr78-mediated M2 macrophage polarization. Similar changes in the levels of M2 markers, such as Arg1, CD206, CD163, IL-10, and CD206, upon treatment with lactate-depleted TCM of human tumor cells (TCM+OA) and following addition of exogenous lactate to TCM (TCM+OA+LA) were observed in parental and OR51E2-depleted THP-1 immortalized human monocytes (FIGS. 5A-G).

In addition, the present inventors examined the effect of acetate, the other short-chain fatty acid that acts as a ligand of Olfr78, on M2 polarization of macrophages. Similar to lactate, acetate treatment significantly (P < 0.001) increased the mRNA levels of M2 markers such as IL-4, IL-10, and TGF-β in WT BMDMs, but treatment with butyrate, which does not interact with Olfr78, did not (FIGS. 4H-J). Acetate did not induce M2 polarization of Olfr78^{-/-} BMDMs (FIGS. 4H-J), suggesting that Olfr78 mediates both lactate- and acetate-induced M2 polarization of macrophages.

### <Example 3> Olfr78 cooperates with Gpr132 to mediate lactate-induced M2 polarization of macrophages

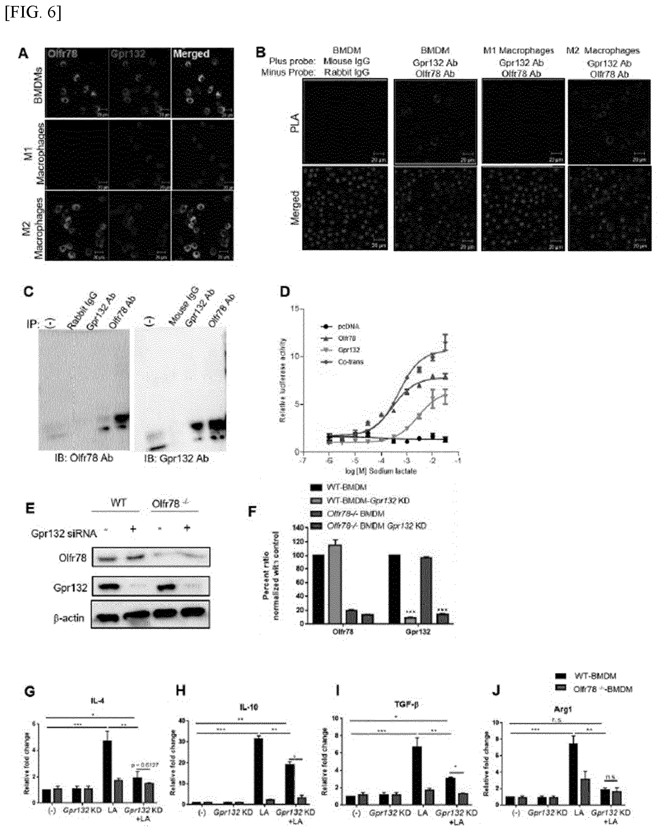

Gpr132 was reported to detect lactate and mediate M2 polarization of macrophages. The present inventors hypothesized that Olfr78 and Gpr132 function together to mediate lactate-induced M2 polarization of macrophages. To investigate this, the present inventors examined the colocalization of Olfr78 and Gpr132 in M1 and M2 macrophages and BMDMs. Gpr132 was expressed in M1 and M2 macrophages and BMDMs (FIG. 6A). By contrast, Olfr78 was expressed in BMDMs and M2 macrophages, where it colocalized with Gpr132, but not in M1 macrophages (FIG. 6A). To investigate the physical proximity of Olfr78 and Gpr132, a proximity ligation assay (PLA) using anti-Olfr78 and anti-Gpr132 was performed. This demonstrated a direct interaction between Olfr78 and Gpr132 on the membrane of BMDMs and M2 macrophages, but not of M1 macrophages (FIG. 6B). In addition, Olfr78 was coimmunoprecipitated by an anti-Gpr132 antibody and Gpr132 was coimmunoprecipitated by an anti-Olfr78 antibody (FIG. 6C). Relative luciferase activity upon treatment with sodium lactate was significantly higher in Hana3A cells cotransfected with Olfr78 and Gpr132 than in those transfected with Olfr78 or Gpr132 alone (P < 0.05) (FIG. 6D). Taken together, these results suggest that Olfr78 and Gpr132 form a functional heterodimer.

To investigate whether Olfr78 and Gpr132 cooperate to mediate lactate-induced M2 polarization, the present inventors depleted Gpr132 in WT and Olfr78^{-/-} BMDMs (FIG. 6E and FIG. 6F). Lactate-induced up-regulation of the mRNA levels of M2 cytokines such as IL-4, IL-10, TGF-β, and Arg1 was significantly (P < 0.01) reduced in Gpr132-depleted WT BMDMs (FIGS. 6G-J). The mRNA levels of IL-4, IL-10, and TGF-β were further reduced in Gpr132-depleted Olfr78^{-/-} BMDMs (FIGS. 6G-I). These results suggest that Olfr78 and Gpr132 cooperate to mediate lactate-induced M2 polarization of macrophages.

### <Example 4> Deficiency of Olfr78 inhibits tumor progression and favors antitumor immunity in vivo

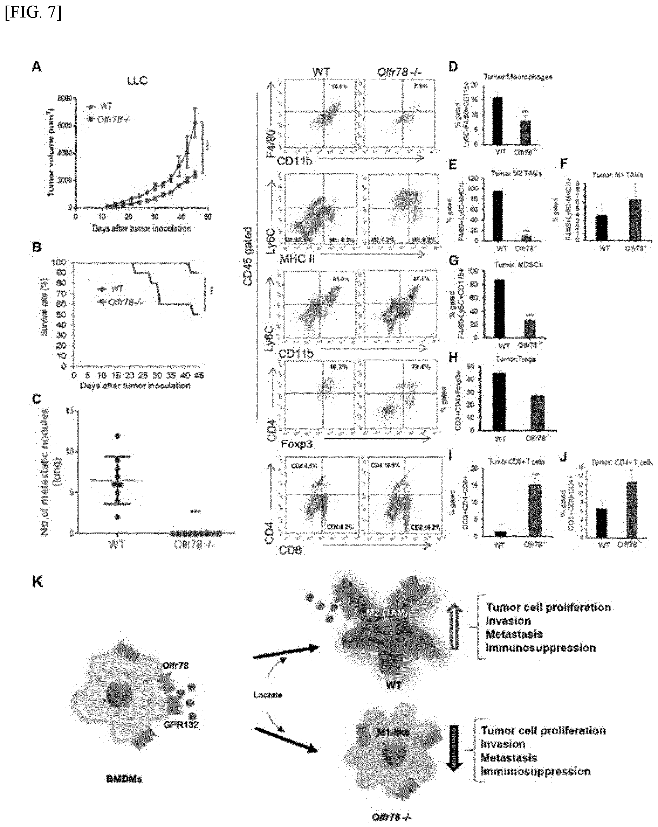

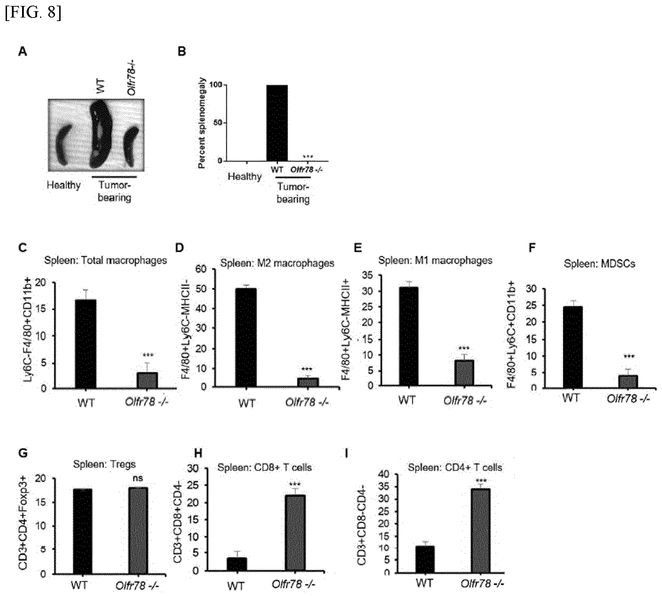

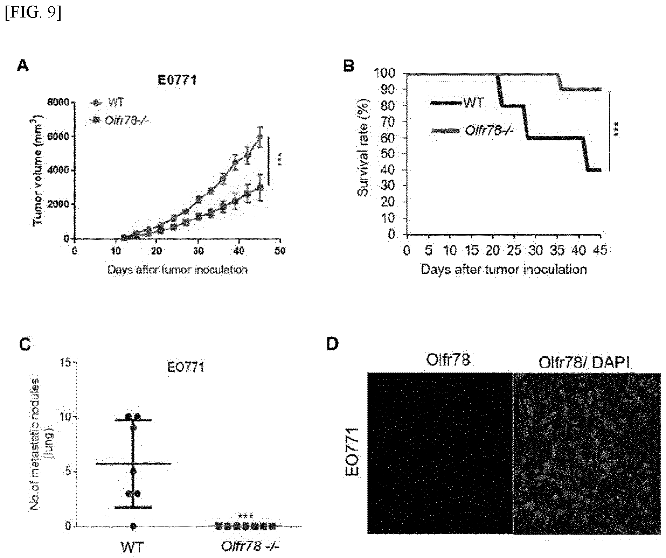

To examine the involvement of Olfr78 in tumor progression in vivo, the present inventors established syngeneic lung tumors in WT and Olfr78^{-/-} mice. Upon subcutaneous implantation of Lewis lung carcinoma (LLC) cells, tumor growth was significantly (P < 0.001) slower (FIG. 7A), the survival rate was significantly (P < 0.001) better (FIG. 7B), and the number of metastatic nodules in the lung was significantly (P < 0.001) lower (FIG. 7C) in Olfr78^{-/-} mice than in WT mice. The populations of total macrophages (FIG. 7D) and immunosuppressive cells including CD45+Ly6C-MHCII-M2-polarized TAMs (FIG. 7E), CD45+Gr1+CD11b+ myeloid-derived suppressor cells (MDSCs) (FIG. 7G), and CD3+CD4+Foxp3+ Tregs (FIG. 7H) were significantly (P < 0.001) smaller in tumor tissues of Olfr78^{-/-} mice than in tumor tissues of WT mice. On the other hand, the populations of immunostimulatory cells including CD45+Ly6C-MHCII+ M1-polarized macrophages (FIG. 7F), CD3+CD4-CD8+ T cells (FIG. 7I), and CD3+CD4+CD8-T cells (FIG. 7J) were larger in tumor tissues of Olfr78^{-/-} mice than in tumor tissues of WT mice. Moreover, tumor-bearing WT mice developed splenomegaly, a condition in which the spleen is enlarged. However, the spleens of tumor-bearing Olfr78^{-/-} mice were similar to those of healthy mice (FIG. 8A and FIG. 8B). The populations of total macrophages (FIG. 8C), M2-TAMs (FIG. 8D), M1 macrophages (FIG. 8E), MDSCs (FIG. 8F), except Tregs (FIG. 8G) were smaller, while those of CD8+ T cells (FIG. 8H) and CD4+ T cells (FIG. 8I), were larger, in spleens of Olfr78^{-/-} mice than in spleens of WT mice. Similar to LLC tumors, upon implantation of EO771 syngeneic mouse breast tumor cells into mammary fat pads, tumor growth was significantly (P < 0.001) slower (FIG. 9A), the survival rate was significantly (P < 0.001) better (FIG. 9B), and the number of metastatic nodules in the lung was significantly (P < 0.001) lower (FIG. 9C) in Olfr78^{-/-} mice than in WT mice. OR51E2 is highly expressed in prostate cancer patients, prostate cancer cells, and several normal cell types. To exclude the possibility that lactate affected tumor growth through Olfr78 on tumor cells, the present inventors measured expression of Olfr78 in EO771 tumors. Olfr78 expression was negligible (FIG. 9D). These results suggest that Olfr78 plays a crucial role in maintenance of an immunosuppressive TME and is thereby a key player in tumor progression and metastasis in vivo.

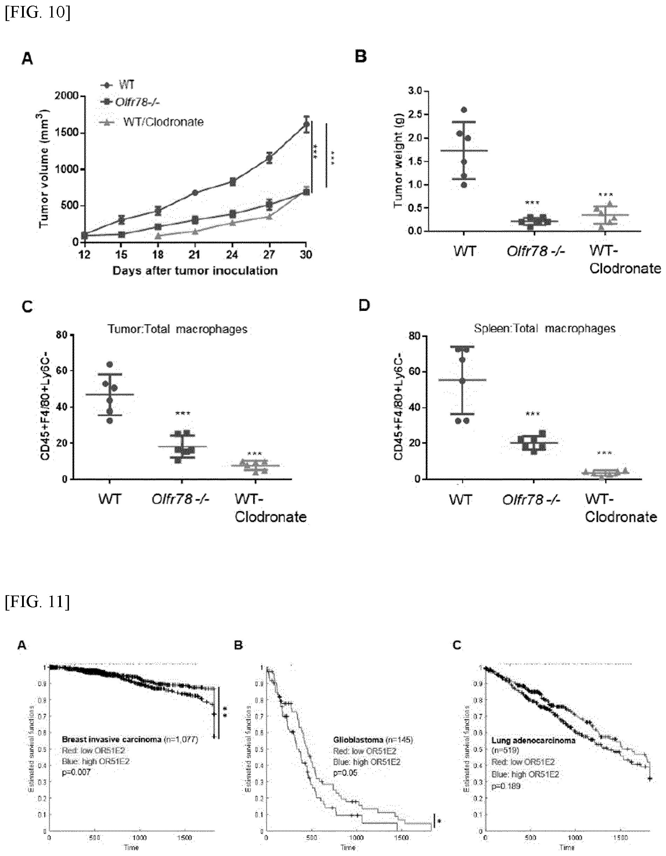

To further examine the involvement of macrophages in tumor progression, the present inventors depleted macrophages using clodronate liposomes in LLC tumor-bearing mice. Compared to Olfr78^{-/-} mice, depletion of macrophages in tumor-bearing mice decreased the tumor volume (FIG. 10A), tumor weight (FIG. 10B), population of total macrophages in the tumor (FIG. 10C), and population of total macrophages in spleen (FIG. 10D).

To determine the clinical significance of OR51E2 in several cancers such as invasive breast carcinoma, glioblastoma, and lung adenocarcinoma, the present inventors searched The Cancer Genome Atlas (TCGA) for treatment analysis reports, gene expression data, and survival rate. Based on patient survival data in TCGA, it was determined that lower OR51E2 expression significantly correlated with longer Kaplan-Meier survival (FIGS. 11A-C). Taken together, these results suggest that perturbation of the Olfr78-lactate interaction may block metastasis in vivo and improve survival rate by impairing M2 macrophage activation.

Having described in detail a particular part of the present invention above, it will be apparent that, for those skilled in the art, such a specific description is merely a preferred example embodiment, and the scope of the present disclosure is not limited thereby. Thus, the substantive scope of the present disclosure will be defined by the appended claims and their equivalents.

## Claims

1. A pharmaceutical composition for preventing or treating cancer, comprising an expression or activity inhibitor of odorant receptor Olfr78 or OR51E2 as an active ingredient.

2. The pharmaceutical composition of claim 1, wherein the expression inhibitor of Olfr78 or OR51E2 is any one selected from the group consisting of antisense nucleotides, small interfering RNAs (siRNAs), and short hairpin RNAs (shRNAs) that complementarily bind to mRNA of Olfr78 or OR51E2 gene.

3. The pharmaceutical composition of claim 1, wherein the activity inhibitor of Olfr78 or OR51E2 is any one selected from the group consisting of low molecular weight compounds, peptides, peptide mimetics, aptamers, antibodies, and natural products that specifically bind to Olfr78 or OR51E2 protein.

4. The pharmaceutical composition of claim 1, wherein the composition inhibits lactate- or acetate-induced M2 polarization of macrophages.

5. The pharmaceutical composition of claim 1, wherein the cancer is lung cancer or breast cancer.

6. A method of screening an anticancer drug, the method comprising:
(1) contacting test substances with isolated macrophages;
(2) measuring a degree of expression or activity of odorant receptor Olfr78 or OR51E2 in the macrophages contacted with the test substances; and
(3) selecting a test substance with a reduced degree of the expression or activity of Olfr78 or OR51E2 compared to a control sample.

7. A method of screening an anticancer drug, the method comprising:
(1) contacting test substances with isolated macrophages;
(2) measuring a degree of binding of odorant receptor Olfr78 or OR51E2 and Gpr132 in the macrophages contacted with the test substances; and
(3) selecting a test substance with a reduced degree of the binding of Olfr78 or OR51E2 and Gpr132 compared to a control sample.

8. The method of claim 6 or 7, wherein the test substance inhibits lactate- or acetate-induced M2 polarization of macrophages.

9. The method of claim 6 or 7, wherein the anticancer drug is an anticancer drug for lung cancer or breast cancer.

10. A reagent composition for inducing lactate- or acetate-induced M2 polarization of macrophages in vitro, comprising odorant receptor Olfr78 or OR51E2 as an active ingredient.

11. A method of inducing lactate- or acetate-induced M2 polarization of macrophages, comprising treating macrophages with odorant receptor Olfr78 or OR51E2 in vitro.
